# EUROPEAN PATENT SPECIFICATION

(11) **EP 1 043 979 B1**
(45) Date of publication and mention of the grant of the patent: **23.01.2002**
(21) Application number: 98962674.2
(22) Date of filing: 21.12.1998
(51) Int. Cl.: A61K 9/70, A61K 47/14

(54) **COMPOSITIONS FOR THE TRANSDERMAL AND DERMAL ADMINISTRATION OF BIOLOGICALLY ACTIVE AGENTS**
ZUSAMMENSETZUNGEN ZUR TRANSDERMALEN UND DERMALEN VERABREICHUNG VON BIOLOGISCH WIRKSAMEN STOFFEN
COMPOSITIONS DESTINEES A L'ADMINISTRATION TRANSCUTANEE ET CUTANEE D'AGENTS BIOLOGIQUEMENT ACTIFS

(30) Priority: 23.12.1997 IT RM970808
(43) Date of publication of application: 18.10.2000
(73) Proprietor: PULITZER ITALIANA S.r.l., 00156 Roma (IT)
(72) Inventor: BERTONE, Evaristo, I-00156 Roma (IT)
(74) Representative: Banchetti, Marina
(86) International application number: IT9800374
(87) International publication number: WO9932094

(56) References cited:
- EP-A- 0 737 477
- US-A- 5 314 694
- US-A- 5 612 382
- SANTUS G C ET AL: "TRANSDERMAL ENHANCER PATENT LITERATURE" JOURNAL OF CONTROLLED RELEASE, vol. 25, no. 1 / 02, 27 May 1993, pages 1-20, XP000361364

## Description

The present invention concerns compositions for the transdermal and dermal administration of biologically active agents. More specifically, the invention relates to formulations containing specific combinations of adjuvants, suited to increase the absorption of therapeutically and cosmetically active agents both in the cutaneous tissues, i.e. at a topical level, and through the skin, i.e. at a systemic level.

As it is known, the administration of pharmaceutically active agents by the transdermal route has been the object of an intense research activity at the end of the sixties, in view of the potential advantages that said route offers in comparison with the conventional systemic administration routes, such as the parenteral route and, especially, the oral route. In contrast to intravenous administration, transdermal administration is clearly not traumatising and does not require the intervention of specialised medical personnel. Differently from oral administration, on the other hand, the transdermal route allows to reduce the variability in the absorption rates with time and from one patient to another, and offers a controlled and sustained release of the drug in the bloodstream. Quite advantageously, moreover, transdermal administration avoids the passage of the drug in the gastrointestinal tract, thus eliminating any possible adverse reaction due to gastric or intestinal intolerance to the drug. In addition, hepatic first pass metabolism is avoided. thus avoiding that the biological activity of the drug be lost - to a major or minor extent depending on the nature of the drug itself - due to metabolic breakdown.

The transdermal delivery devices or transdermal therapeutic systems (TTS) (i.e., transdermal administration forms employing particular plaster or patch structures) that have first attained the commercial stage include those for the controlled release of nitroglycerin and other nitro derivatives, used for the treatment and the prophylaxis of angina pectoris and of other cardiovascular conditions, and those for the administration of estradiol and other estrogens as well as progestogens, and for the administration of hormones in general. Also widespread are the systems for transdermal administration of antiemetics, for use in the therapy and the prevention of motion-sickness, and those for the administration of agents against nicotine addiction. In spite of the cited advantages, however, the number of products for transdermal administration presently on the market is limited, mainly due to the practical difficulties connected with the mechanism of drug permeation through the skin. The passage of an active substance through the complex membrane formed by the skin includes the passage through the external stratum corneum, the diffusion through the epidermis and the papillar dermis and, finally, the penetration through the walls of capillary blood or lymph vessels. This is clearly a complex phenomenon, that encounters different resistances in each kind of tissue. It is, however, the stratum corneum, with its dense layer of highly keratinised cells, that represents the primary barrier opposing to the absorption of external substances in the skin, or to the passage of said substances through the skin.

Most active agents that are being tested for transdermal administration would not penetrate through the skin, or would penetrate to an extent unsuitable for therapeutic application, were they not accompanied by special adjuvants known as "penetration enhancers" or "permeation enhancers" or, less frequently, "absorption promoters". The said substances, by acting through various and sometimes unknown mechanisms, help the active ingredient to overcome the barrier of stratum corneum thus enhancing the transdermal flux, i.e. the amount of drug permeating through the skin per application area unit and per time unit.

The first works on transdermal permeation enhancers concern dimethylsulphoxide (DMSO) and N,N-dimethylacetamide (DMA) (US Patents No. 3,551,554, to Crown Zellerbach, and No. 3,472,931, to Foster Milburn, both of 1970). These compounds, together with other solvents such as dimethylformamide (DMF) and ethanol, increase the penetration through the skin as a result of their ability to solubilise the stratum corneum lipids. In spite of their remarkable initial popularity, DMSO and DMA are being gradually abandoned as penetration enhancers, owing to their irritative action on the skin and to their potential toxicity. In their place, higher molecular weight analogues have been proposed, which are expected to have a better tolerability, such as, e.g., decylmethyl sulphoxide (C₁₀MSO), or long chain aliphatic amides (see, e.g., US Patent No. 4,999,379, to Ciba Geigy).

Such first group of penetration enhancers also includes ethanol, that has been proposed for the transdermal administration of estradiol since 1983 (US Patent No. 4,379,454 and others, to Alza). The high permeation capacity of ethanol, in addition to causing problems of skin tolerability, required the use of excessive amounts of ethanol in order to avoid that the transdermal system be depleted of ethanol before the active ingredient was used up. For this reason, a TTS with a separate reservoir containing enhancer and drug in the form of a solution had to be used, wherein a rate-controlling membrane is placed between the reservoir and the skin on which the system is adhered. The membrane is freely permeable to the drug but is rate limiting for the enhancer, so as to properly limit the ethanol flux.

Among the several products that have thereafter been proposed for use as permeation enhancers there may be cited, in addition to ethanol and isopropyl alcohol, long chain aliphatic alcohols (e.g., US Patent No. 4,906,169, to Rutgers Univ.; PCT application publ. No. 90/04397, Schering Plough), multifunctional alcohols such as propylene glycol or glycerol, often in combination with ethanol in order to reduce its irritating power (e.g., US Patent No. 4,855,294, to Theratech), fatty acids, widely employed both alone and in combination with other enhancers (e.g., US Patent No. 4,626,539, to Du Pont de Nemours; European Patent No. 255485, to Warner Lambert, in particular on linoleic acid; US Patent No. 4,863,970, to Theratech, in particular on oleic acid and corresponding alcohols, in combination with a lower alcohol), and fatty acid esters. Among the latter, isopropyl myristate has been preferred, in view of its current use as an excipient in many pharmaceutical and cosmetic preparations, the good dermal tolerability of which is ascertained (e.g., European Patent No. 436203, to Nitto).

Also preferred as penetration promoters are lauric acid and lauric acid esters, that are considered to be particularly suitable as enhancers in view of the number of carbon atoms of their aliphatic chain, C₁₂. The chain length is such as to confer on the molecule sufficient lipophilic properties to interact with the stratum corneum lipids, but also sufficient hydrophilic properties to interact with the protein structure. Examples of patent documents on this issue are US Patent No. 4,568,343 (Alza), on polyethylene glycol monolaurate (PEGML), US Patent No. 4,746,515 (Alza), on glyceryl monolaurate (GML), European Patent No. 272987 (Cygnus), on propylene glycol monolaurate (PGML), as well as US Patent No. 4,537,776 (Procter & Gamble), concerning a combination of methyl laurate and N-2-hydroxyethyl pyrrolidone.

Also N-methyl-2-pyrrolidone and the derivatives thereof, such as the one mentioned above, have received much attention as penetration enhancers. However, also in this case the toxicity of the molecule is such as to discourage their use at the concentration levels that would be necessary for their action as permeation enhancers. A derivative subsequently proposed on the basis of a lower toxicity is 3-hydroxy-N-methyl-2-pyrrolidone (US Patent No. 5,032,402).

Other groups of compounds that have been found to be active in promoting the transdermal penetration of various active ingredients are Azone® (1-n-dodecylazacycloheptan-2-one) and the derivatives thereof (US patents to Nelson, Nos. 3,989,816, 4,316,893 and others), the mechanism of action of which has not yet been fully clarified; essential oils and the components thereof such as, e.g., menthol, eugenol, eucalyptol (US Patent No. 4,560,553, to Merck) and various terpenoids, the use of which is well proven both in the cosmetic and in the pharmaceutical filed, thus offering less toxicity risks; phospholipids and phosphate derivatives, such as, e.g., lecithin (US Patent No. 4,783,450, to Warner Lambert), and sorbitan mono-, di- and triesters of fatty acids, optionally in combination with lower aliphatic alcohols (US patent No. 5,122,383, to Theratech).

Also fatty acid alkanolamides (i.e., N-hydroxyalkyl- or N-di(hydroxyalkyl)amides) have been proposed as penetration enhancers, although no commercial transdermal delivery systems using alkanolamides as enhancers are known at present. For instance, European Patent No. 0083371 (Key Pharmaceuticals), concerning vehicles for the transdermal administration of nitroglycerin, proposes a diffusion matrix for TTS containing the active ingredient together with a polymer mixture and, preferably, an alkanolamide of a C₈-C₁₈ acid. The presumed function of the latter surface active ingredient is to enhance transdermal absorption. The European Patent No 0196679 (Rutgers Univ.), concerning a polymeric matrix-type dosage unit for the transdermal administration of medicaments, proposes fatty acid diethanolamides among the possible enhancers to be included in the TTS. Further, PCT application publ. No. 94/21262 (Alza) discloses a transdermal delivery system for an antianxiety medicament, alprazolam, wherein the preferred permeation promoters are fatty acid diethanolamides.

Another document disclosing the use of fatty acid alkanolamides (in particular, lauric diethanolamide) as penetration enhancers is PCT application publ. No. 94/15609 (Sunkyong Industries). The latter proposes to employ, in addition to the proper enhancers, some adjuvant agents referred to as "absorption assistants", which are expected to improve the enhancer performance by increasing the solubility of the active ingredient. The said absorption assistants are solvents with both hydrophilic and lipophilic properties, which favour and regularise absorption and permeation of both the active ingredient and the enhancer; and allow to increase the concentration of active ingredient in the matrix. Some of the absorption assistants cited in the concerned document are the solvents already mentioned in the foregoing in connection with their activity as penetration enhancers (i.e., DMSO, DMA, DMF, N-alkylpyrrolidone), whose topical toxicity is ascertained. In their role of "absorption assistants", such agents are to be included in the formulation in sufficiently diluted form to have a toxicity below the acceptable limits. This implies a reduction of their action as enhancers below the useful levels, although their solvent properties are maintained to a level sufficient for their activity as absorption assistants.

Although the above considerations are more directly referred to the field of transdermal delivery, wherein a suitable formulation containing a pharmacologically active agent is applied to an area of intact skin in order to obtain penetration of the active ingredient through the surface tissues up to the bloodstream, it is evident that quite similar problems and solutions attach to systemic transmucosal administration, for example the administration through the sublingual route. Analogy aspects may also be found in the topic dermal administration, wherein the drug is required to penetrate the stratum corneum and/or the superficial layers of the affected or injured dermal area so as to be available in an optimal concentration in the affected cutaneous tissues. Further, analogies are found in the administration of biologically active ingredients in the cosmetic field, where the cutaneous penetration is a major object.

In the light of the above prior art, it is an object of the present invention to provide an improved formulation capable of enhancing cutaneous absorption of known therapeutically and cosmetically active ingredients. Such formulation is intended to exploit transdermal permeation enhancers chosen from the group of high molecular weight alkanolamides, potentiated by absorption assistants that are neither toxic nor irritant, and are capable of considerably increasing the transdermal flux of the concerned active ingredient through the skin.

Within the frame of the studies that led to the present invention, the main known penetration enhancers (either already at the commercial stage or more recently disclosed) have been considered, such as, e.g., fatty acids and fatty alcohols of higher molecular weight, polyhydroxy alcohol esters of fatty acids, sorbitan esters, etc.. The attention has been concentrated on a feature considered to be of the utmost importance for the performance of a compound as permeation enhancer, i.e. the balance of hydrophilic and lipophilic properties of the said compounds. The HLB value (i.e., hydrophile-lipophile balance) of the various products was calculated according to the known methods (e.g., according to Voigt, Rudolf: Lehrbuch der pharmazeutischen Technologie, 1979, 352) and then confirmed, as customary, with analytical data such as saponification number, acid number, solubility, dispersibility, etc.. It has thus been ascertained that the HLB value of the studied compounds is comprised between 1.5 and 7.5.

Many alkanolamides have a HLB value comprised within said range; however, in particular, higher molecular weight monoethanolamides and diethanolamides have HLB values falling in the middle-lower section of the cited range. In order to increase the HLB value of the studied compositions to optimal levels, the addition of suitable amounts of compounds with higher HLB has been considered, according to a known formulation method. The specific choice of the agents thus added to increase the HLB value led to the finding of some combinations that show a superior activity in promoting the transdermal absorption of active ingredients of various nature.

It has thus been found, according to the present invention, that combining a penetration enhancer belonging to the class of fatty acid alkanolamides with an adjuvant chosen from the group of low molecular weight aliphatic acid alkanolamides, i.e. C₁-C₄ alkanolamides (which are endowed with a sufficiently high HLB value to bring the overall HLB to optimal levels) it is possible to obtain formulations for transdermal delivery with a remarkably improved skin penetration. The permeation of pharmacologically active substances or cosmetic active agents through cutaneous tissues is thus most efficiently assisted. It is to be noted that the addition of many other substances with a high HLB value, even if these are compatible with the other ingredients of the transdermal system and are pharmaceutically acceptable, may involve other drawbacks that make such addition objectionable. Such is the case, e.g., of ionic surfactants, that are unsuitable in that their HLB value, depending on the ionisation degree, is extremely variable. Such is also the case of nonionic surfactants, generally oxyalkylene derivatives, that may negatively affect the release of the drug by increasing the affinity thereof for the TTS matrix.

The improved performance of the compositions according to the invention as compared with compositions containing only fatty acid alkanolamides as penetration enhancers can be interpreted not only in terms of an optimisation of the hydrophile-lipophile balance, but also considering that aliphatic acid ethanolamides with one to four carbon atoms play in the dermal or transdermal preparation an active role as absorption assistants. This action is similar to that disclosed in the above cited PCT application publ. No. 94/15609. N-ethanolacetamide, for instance, has the same hydrophilic-lipophilic solvent properties as the products proposed by the said prior art document, but, in addition, it has the advantage of not being toxic nor irritant. Actually, it has been shown that ethanolamides have an antiinflammatory and antiallergic activity (F. A. Kuehl et al., J. Am. Chem. Soc., 79, 5577, 1957). The cited authors, after isolating N-(2-hydroxyethyl)palmitamide from natural sources and upon examining other synthetic ethanolamides, ascertained that the pharmacological properties of such compounds are due exclusively to the basic portion of the molecule. More specifically, such activity is selectively shown by the group = N - CH₂ CH₂OH, as the higher omologues, i.e. N-(2-hydroxypropyl)amides and N-(3-hydroxypropyl)amides, are not active.

Thanks to their antiallergic and antiinflammatory action, and to the absence of any topical toxicity, the low molecular weight alkanolamides provided as absorption assistants according to the invention can be included in the formulation in sufficiently high concentrations. There is no need to excessively dilute the said agents, as it happens with the inherently toxic DMSO, DMF and other agents that have been proposed for use as absorption assistants. As a result, the solvent power and the antiinflammatory action of the low molecular weight alkanolamides proposed may be exploited at best, especially in the preparations normally offering some risks of adverse side effects, e.g. in terms of cutaneous inflammation, such as occlusive transdermal patches.

Accordingly, the present invention specifically provides a composition for the transdermal and dermal administration of biologically active agents comprising an effective amount of one or more biologically active agents, one or more penetration enhancers chosen from the group consisting of monoalkanolamides and dialkanolamides of carboxylic acids having at least 8 carbon atoms, wherein the hydroxyalkyl groups contain from 2 to 21 carbon atoms, one or more absorption assistants chosen from the group consisting of monoethanolamides and diethanolamides of carboxylic acids having from 1 to 4 carbon atoms and, optionally, further biologically acceptable permeation adjuvants and excipients.

The alkanolamides employed in the formulation according to the invention as permeation enhancers have the following structural formula: 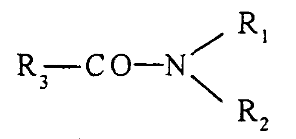 wherein each R₁ and R₂ independently represents hydrogen or a hydroxyalkyl group, i.e. - (CH₂)ₙOH, wherein n is an integer from 2 to 21, with the provido that at least one of R₁ and R₂ is a hydroxyalkyl group, and R₃ is a hydrocarbyl group having at least 8 carbon atoms, preferably a mono-unsaturated, di-unsaturated or tri-unsaturated aliphatic group having 8-22 carbon atoms, optionally substituted with from 1 to 3 hydroxy groups.

According to a preferred embodiment of the composition according to the invention, the monoalkanolamide or dialkanolamide of the penetration enhancer is, respectively, a monoethanolamide or a diethanolamide. The latter, as pointed out in the foregoing, are non-topically irritant products showing, on the contrary, a marked antiallergic and antiinflammatory activity. Examples of alkanolamides of high molecular weight aliphatic acids that may be advantageously employed as enhancers in the formulation according to the invention are N-(2-hydroxyethyl)amides or N,N-di(2-hydroxyethyl)amides of caprilic, caprinic, lauric, myristic, palmitic, stearic, oleic, linoleic, linolenic and undecylenic acids.

As the preferred absorption assistants the invention proposes to employ N-(2-hydroxyethyl)acetamide or N,N-di(2-hydroxyethyl)acetamide. As noted before in respect of all ethanolamides, these compounds are particularly suitable in view of their activity against any occurrence of cutaneous irritation. Mixtures formed by N-(2-hydroxyethyl)acetamide, which is a water-soluble and liposoluble high boiling point liquid (151-155°C), and the fatty acid monoethanolamides or diethanolamides mentioned above show HBL values comprised in the optimal range of 4 to 7.

In addition to fatty acid alkanolamides, serving as penetration enhancers, and to alkanolamides of low molecular weight acids, serving as absorption assistants, the formulation may also include other promoters, as well as, in particular, aliphatic alcohols chosen from the group consisting of ethanol, n-propanol, isopropanol, buthanol and mixtures thereof, in suitably diluted amounts, serving as co-solvents.

The delivery methods that may be used for the compositions according to the invention are many, as it is possible to administer such compositions, e.g., in the form of creams, ointments, gels, suspensions, emulsions, etc.. The form will depend on the choice of the excipients, and the formulation techniques are well known both in the cosmetic and in the pharmaceutical field. In the case of pharmaceutical products, in particular, to the therapeutic dose of active ingredient there will be added an amount from 0.5% to 40% by weight of the penetration enhancers according to the invention, and an amount from 0.1% to 40% by weight of the absorption assistants according to the invention, based on the total weight of the composition. The latter may also contain, for instance, preservatives, antioxidant agents, gelling agents, thickeners, surfactants, stabilisers, plasticisers and the like, obviously chosen from the ingredients suitable for use in pharmaceutical products for topical administration.

According to some preferred embodiments of the invention, the claimed composition is presented in a transdermal therapeutic system (TTS), which may have one of the different known structures that have been proposed for such administration forms. Specifically, the TTS may be of the liquid reservoir type (or first generation type) wherein the drug and the possible enhancer are placed, optionally together with other excipients, in the cavity of a patch structure closed, on the side intended to be adhered to the skin, by a porous membrane (as well as by the required layer of adhesive). The presently preferred type of TTS is, however, the so-called matrix type system, wherein the active ingredient is incorporated in a solid matrix. In the most recent version, such system consists of a simple three-layer laminate having a first backing layer, a second layer consisting of the matrix containing the active ingredient together with the optional enhancers and excipients and a pressure sensitive adhesive, and a third removable protective layer (i.e. release liner), to be discarded before use. This form of TTS is presently the most widespread one owing to the advantages it offers from the standpoint of simple construction and cheap industrial production, and owing to its reduced size, resulting in a better patient compliance.

According to a further aspect thereof, the invention thus provides a system for transdermal administration of pharmaceutically active agents consisting of a laminate comprising:
(a) a backing layer impermeable to the constituents of the adjacent layer (b);
(b) a matrix layer consisting of a solid mixture containing an effective amount of one or more pharmaceutically active agents, one or more penetration enhancers chosen from the group consisting of monoalkanolamides and dialkanolamides of carboxylic acids having at least 8 carbon atoms, wherein the hydroxyalkyl groups contain from 2 to 21 carbon atoms, one or more absorption assistants chosen from the group consisting of monoethanolamides and diethanolamides of carboxylic acids having from 1 to 4 carbon atoms and one or more pressure-sensitive adhesives, as well as further biologically acceptable permeation adjuvants and excipients;
(c) a removable protective layer, to be peeled from said matrix layer before use.

The preferred choices for the penetration enhancer and the absorption assistant are the same as set forth above. Preferably the TTS comprises the said agents in the following concentrations: from 2.5% to 15% by weight of penetration enhancer and from 0.2% to 20% by weight of absorption assistant.

In special cases recourse may be had to a multilayer TTS, including more matrix layers of different compositions.

The backing layer of the transdermal system according to the invention consists, in accordance to the known art, of a polymeric film, which must be flexible and soft, chemically inert and impermeable to the active ingredient and to the other components of the adjacent layer. The backing strongly adheres to the matrix and, after peeling off the release liner, allows to handle the said matrix so as to correctly apply it onto the skin. In addition, the impermeable backing renders the administration occlusive. Examples of polymers that may be employed for the backing layer are polyethylene, polyesters, polyurethanes, polypropylene and the like. The preferred material is low density polyethylene (available from 3M).

In addition to the active ingredient, the enhancer, the absorption assistant and other possible adjuvants, the matrix contains a pressure-sensitive adhesive (or contact adhesive), which should be chosen from the products suitable for a prolonged contact with the skin and capable of providing, along with the stability of adhesion, the continuity of transdermal administration. Further, the adhesive must be physically and chemically compatible with all other constituents of the matrix, and must incorporate said constituents by dissolving them at least partially, while maintaining its adhesive power and its consistency, in spite of the unavoidable dilution thereof. Examples of suitable adhesives are polysiloxanes, polyurethanes, polyacrylates, polyisobutylene, hydrogenated colofony. Polyacrylates with various degrees of cross-linking are preferred, such as those produced for use in transdermal devices, e.g. with the trade names Durotak® (National Starch Chemical Co.), Gelva® (Monsanto) and Plastoid® (Röhm Pharma). Particularly preferred are the acrylate adhesives marketed as Durotak® 387-2825 and Durotak® 387-2052, both characterised by the presence of acid groups (-COOH) in the molecule, and by the presence in the mixture of a cross-linking system formed by a cross-linking agent (i.e. catalyst) and by a stabiliser. The cross-linking system is activated during the manufacture of the patch.

The protective layer consists of a polymer which is impermeable and inert to the active ingredient and to all of the other components of the matrix. Such layer must be readily removable from the matrix before application of the patch. The best results have been obtained by silanising or, better, fluorinating the surface of the protective polymer. Polyester films fluorinated on one or on both sides (available from 3M) are preferred.

The transdermal systems described above may be produced according to standard methods, such as, e.g., by forming a thin layer (i.e., the matrix) starting from a suitably concentrated solution and evaporating the solvent. The matrix can be thus formed either on the backing layer or on the protective layer. The further layers are then laminated on the two-layer structure so obtained.

The transdermal and dermal administration compositions and systems according to the invention may be used, in the medical field, with several pharmaceutically active agents, among which there are mentioned, by way of example, antianginal and coronary vasodilator agents (such as nitroglycerin, isosorbide mononitrate and isosorbide dinitrate), antiarrythmics, cardiovascular and antihypertensive agents, including beta-blockers (such as pindolol and timolol) and calcium-channel blockers (such as verapamil and nifedipine); steroids, in particular estrogens and progestogens (such as estradiol, progesterone, norethisterone or norethindrone, medroxyprogesterone), rubefacients and blood flow stimulating agents (such as nicotinic derivatives, vanillylamide of pelargonic acid or nonivamide, and capsaicin), antiemetics and agents for the therapy of motion-sickness (such as scopolamine), agents against smoke addiction (such as nicotine), antiasthmatics (such as formoterol), non steroidal antiinflammatory and antirheumatic agents (such as indometacin, piroxicam, diclofenac), analgesics (such as morphine) and, moreover, antiinfective agents, antibiotics, antiviral agents, antihelminthics, antiarthritics, antipyretics, anticonvulsants and bronchodilators, decongesting agents, antihistamines and antipruritics, sedatives and anxiolytics, antipsychotics, antidepressants, cholinergics, central nervous system stimulating agents, antidiabetics, antidiarrheals, antineoplastics, antiparkinsonians, immunosuppressants. According to some specific embodiments of the invention, the said pharmaceutically active agents are chosen from the group consisting of: antianginal and coronary vasodilating agents, steroids, in particular estrogens and progestogens, and rubefacient and blood flow stimulating agents.

In view of what noted above as concerns the possibility of using the compositions according to the invention for the topical treatment of dermatological affections, the claimed compositions, containing the proper active ingredients, can also be used as antifungals, topical antibiotics and chemotherapeutics, reepithelising agents, anticellulitis agents, moisturisers, keratolytic and peeling agents, exfoliating agents, vitamin products and the like.

In the course of the experimentation carried out with the preferred compositions according to the invention, in particular for the transdermal administration of nitroglycerin, it has been found that the appearance of the release peak is delayed, shifting, e.g., from the 7^{th}-9^{th} hour to the 11^{th}-13^{th} hour on a total application time of 24 hours. At the same time, the overall release values are maintained at substantially constant levels. It is evident that such modulating effect on the drug release, which can be ascribed to the combination of fatty acid alkanolamides with a low molecular weight ethanolamide characterising the claimed composition, can avoid the appearance of excessive blood concentrations of the active ingredient, thus avoiding any connected side effects. Also, the said modulating effect can afford a higher therapeutic efficiency, especially when the active ingredient has a very short half-life. This effect is particularly important for the matrix-type transdermal devices consisting of three-layer laminates, that lack any rate-controlling membrane and are characterised by a high concentration of the active ingredient in the matrix.

Some specific embodiments of the invention are described below for merely illustrative purposes, together with the results of the experimental studies carried out on the proposed formulations, and to the comparative tests with other transdermal penetration enhancer compositions.

### EXAMPLE 1

### TTS for the administration of nitroglycerin

The transdermal delivery devices employed in this example and in the following ones were produced according to the following procedure. A matrix layer containing the active substance, the adhesive and, when present, the permeation enhancer and the absorption assistant was obtained by uniformly spreading a solution of the components, with the proper concentration, viscosity and quantitative composition, on the silanised or fluorinated surface of a polyester protective layer. After evaporating the solvent at 70-80°C for 15-20 min. a final layer of a homogeneous thickness was obtained. weighing about 10 mg/cm² and having the desired composition. The composition had been preliminarily fixed taking into account the amount of solvent to be removed. During the above operation the adhesive employed underwent the required cross-linking reaction. The final three-layer laminate TTS was produced by laminating a layer of low density polyethylene on the double layer obtained from the previous operation.

All samples tested contained 33% nitroglycerin (Di Pharma) and polyacrylate adhesive Durotak® 387-2825 (National Starch Chemical Co.). The nitroglycerin concentration was kept constant in the various preparations, since any changes thereof influence the transdermal flux. In the first system tested no penetration enhancers were added, while the second system contained the known enhancer sorbitan monooleate (Arlacel 80, ICI) by way of comparison. The third system under test contained oleic acid diethanolamide (Henkel) as enhancer, but had no absorption assistant, while the fourth system also contained N-(2-hydroxyethyl)acetamide (Aldrich) as absorption assistant.

On the transdermal devices thus produced *in vitro* release tests were carried out, employing methods well known to this aim (Merrit e. Cooper, J. Controlled Release, 1984, 1, 161), that have been further developed more recently (Reifenrath et al., J. Pharm. Sci., 80, 1991, 526). These methods are carried out on human skin excised from cadaver. The skin, properly pretreated in order to moisturise it, is mounted on specially designed diffusion cells known as Franz cells, with the epidermal side exposed (stratum comeum) and the opposed side kept in contact with a liquid receiving medium. The tested patches were applied to the exposed side of the skin, and the transdermal flux was measured by analysing (by HPLC) the receiving medium. The results thus obtained are expressed in the following table in µg/cm² h, as the average over three tests +/- standard error.

**TABLE 1**

| Transdermal flux of nitroglycerin | | | | |
|---|---|---|---|---|
| Components | Formulation under test | | | |
| | n°.1 | n°.2 | n°.3 | n°.4 |
| nitroglycerin (%) | 33 | 33 | 33 | 33 |
| sorbitan monooleate (%) | 0 | 4 | 0 | 0 |
| oleic diethanolamide (%) | 0 | 0 | 4 | 4 |
| N-(2-hydroxyethyl)acetamide (%) | 0 | 0 | 0 | 2 |
| adhesive | q.s. to 100 | q.s. to 100 | q.s. to 100 | q.s. to 100 |
| transdermal flux (µg/cm² h) | 27.3±2.5 | 38.1±3.2 | 41.0±2.7 | 48.0±3.0 |

The foregoing results show the positive and quantitatively remarkable effect of a combination of high molecular weight and low molecular weight alkanolamides acting, respectively, as penetration enhancer and as absorption assistant, on the transdermal release of nitroglycerin. The beneficial effect of the combination is remarkable not only in comparison with a preparation with no enhancers, but also in comparison with preparations wherein the fatty acid ethanolamide promoter is not accompanied by the absorption assistant of the invention.

### EXAMPLE 2

### TTS for the administration of nitroglycerin

### Comparison with N-(2-hydroxyethyl)acetamide alone

In order to ascertain the possible penetration enhancing effect of N-ethanolacetamide, four transdermal systems were produced, following the preparation method of Example 1 and employing the starting materials cited therein. The TTS contained a constant amount of nitroglycerin as the active ingredient and increasing amounts of N-(2-hydroxyethyl)acetamide, starting from zero. The corresponding transdermal fluxes, measured as shown in Example 1, are reported in the following table.

**TABLE 2**

| Transdermal flux of nitroglycerin - Comparative tests | | | | |
|---|---|---|---|---|
| Components | Formulation under test | | | |
| | n°.1 | n°.2 | n°.3 | n°.4 |
| nitroglycerin (%) | 33 | 33 | 33 | 33 |
| N-(2-hydroxyethyl)acetamide (%) | 0 | 3 | 5 | 7 |
| adhesive | q.s. to 100 | q.s. to 100 | q.s. to 100 | q.s. to 100 |
| transdermal flux (µg/cm² h) | 27.8±1.8 | 26.9±2.5 | 28.2±2.1 | 30.5±2.7 |

From the above experimental results there may be concluded that N-(2-hydroxyethyl)acetamide alone does not possess any significant activity as enhancer. By increasing the concentration, a trend towards increasing release rates is detectable.

### EXAMPLE 3

### TTS for the administration of nitroglycerin

### Comparison with sorbitan monooleate

Some transdermal systems containing increasing amounts of an enhancer of the prior art, i.e. sorbitan monooleate, were prepared according to the method of Example 1. Further, other patches were prepared with the same method, containing the penetration enhancer and the absorption assistant of the invention. The nitroglycerin concentration was kept constant in all preparations, as its variations affect the release. The transdermal fluxes measured with the method of Example 1 are shown in the following tables.

**TABLE 3**

| Transdermal flux of nitroglycerin - Comparative tests | | | | |
|---|---|---|---|---|
| Components | Formulation under test | | | |
| | n°.1 | n°.2 | n°.3 | n°.4 |
| nitroglycerin (%) | 33 | 33 | 33 | 33 |
| sorbitan monooleate (%) | 0 | 3 | 5 | 7 |
| adhesive | q.s. to 100 | q.s. to 100 | q.s. to 100 | q.s. to 100 |
| transdermal flux (µg/cm² h) | 28.1±2.8 | 36.7±3.0 | 40.6±4.2 | 31.5±3.3 |

**TABLE 4**

| Transdermal flux of nitroglycerin | | | | | | |
|---|---|---|---|---|---|---|
| Components | Formulation under test | | | | | |
| | n°.5 | n°.6 | n°.7 | n°.8 | n°.9 | n°.10 |
| nitroglycerin (%) | 33 | 33 | 33 | 33 | 33 | 33 |
| oleic diethanolamide (%) | 3 | 3 | 5 | 5 | 7 | 7 |
| N-(2-hydroxyethyl)acetamide (%) | 0 | 1.5 | 0 | 2.5 | 0 | 3.5 |
| adhesive | q.s. to 100 | q.s. to 100 | q.s. to 100 | q.s. to 100 | q.s. to 100 | q.s. to 100 |
| transdermal flux (µg/cm² h) | 34.7±3.5 | 46.3±3.1 | 43.2±2.4 | 54.1±3.8 | 47.2±4.5 | 57.7±4.0 |

The above results show, in general, that an increase of the enhancer concentration or of the concentration of the enhancer mixture corresponds to an increase in the release. An exception to this rule is represented by sorbitan monooleate. The latter shows, at concentrations above 5-6% and in the specific case of nitroglycerin, a relative reduction of the transdermal permeation, probably due to a higher affinity of the matrix to nitroglycerin in such conditions.

All the compositions containing combinations of high and low molecular weight alkanolamides according to the invention produced transdermal fluxes of nitroglycerin higher than those of the controls with no penetration enhancers, and also higher than those containing the enhancers without absorption assistants.

### EXAMPLE 4

### TTS for the administration of nitroglycerin

The procedure of Example 1 was followed for the preparation of transdermal devices having increasing amounts of lauric monoethanolamide as enhancer, with or without N-(2-hydroxyethyl)acetamide as absorption assistant. The latter was also included in increasing amounts. In this case as well, the nitroglycerin concentration was kept constant. The transdermal fluxes measured are shown in the following table.

**TABLE 5**

| Transdermal flux of nitroglycerin | | | | | | | |
|---|---|---|---|---|---|---|---|
| Components | Formulation under test | | | | | | |
| | n°.1 | n°.2 | n°.3 | n°.4 | n°.5 | n°. 6 | n°.7 |
| nitroglycerin (%) | 33 | 33 | 33 | 33 | 33 | 33 | 33 |
| lauric monoethanolamide (%) | 0 | 3 | 3 | 5 | 5 | 7 | 7 |
| N-(2-hydroxyethyl) acetamide (%) | 0 | 0 | 4 | 0 | 6.6 | 0 | 9.3 |
| adhesive | q.s. to 100 | q.s. to 100 | q.s. to 100 | q.s. to 100 | q.s. to 100 | q.s. to 100 | q.s. to 100 |
| transdermal flux (µg/cm² h) | 27.1±2.2 | 31.0±4.5 | 37.3±3.0 | 35.2±1.9 | 42.3±3.1 | 40.8±3.3 | 46.8±2.9 |

The use of a monoethanolamide as penetration enhancer was associated to a ratio of the latter to the absorption assistant of about 1:1.33, while for diethanolamides such ratio was 1:0.5 (see Examples 1 and 3). The use of a higher amount of assistant for monoethanolamides is in agreement with what previously set forth with regard to the optimal HLB setting.

As it may be noted, by increasing the promoter concentration or the concentration of the promoter-absorption assistant mixture, increasing transdermal flues have been obtained. In all cases the preparations according to the invention produced nitroglycerin releases higher than those of the controls with no enhancers, and also higher than those of the comparative samples containing permeation enhancers with no absorption assistant.

### EXAMPLE 5

### TTS for the administration of nitroglycerin

The experimentation of Example 4 was repeated replacing lauric monoethanolamide with lauric diethanolamide (Henkel), and keeping N-(2-hydroxyethyl)acetamide as absorption assistant. Transdermal fluxes of nitroglycerin higher than from the controls with no enhancers have been obtained, and also higher than from the comparative samples containing permeation enhancers with no absorption assistant.

### EXAMPLE 6

### TTS for the administration of nitroglycerin

The experimentation of Example 4 was repeated replacing lauric monoethanolamide with oleic monoethanolamide (Henkel), and keeping N-(2-hydroxyethyl)acetamide as absorption assistant. Transdermal fluxes of nitroglycerin higher than from the controls with no enhancers have been obtained, and also higher than from the comparative samples containing permeation enhancers with no absorption assistant.

### EXAMPLE 7

### TTS for the administration of nitroglycerin

The experimentation of Example 4 was repeated replacing lauric monoethanolamide with palmitic diethanolamide (Henkel), and keeping N-(2-hydroxyethyl)acetamide as absorption assistant. Also in this case, transdermal fluxes of nitroglycerin higher than from the controls with no enhancers have been obtained, and also higher than from the comparative samples containing permeation enhancers with no absorption assistant.

### EXAMPLE 8

### TTS for the administration of nitroglycerin

The experimentation of Example 4 was repeated replacing lauric monoethanolamide with undecylenic monoethanolamide (Henkel), and keeping N-(2-hydroxyethyl)acetamide as absorption assistant. Transdermal fluxes of nitroglycerin higher than from the controls with no enhancers have been obtained, and also higher than from the comparative samples containing permeation enhancers with no absorption assistant.

### EXAMPLE 9

### Test for possible side effects

The possible side effects (i.e. local reactions) of the formulations under test have been evaluated *in vivo.* By means of calibrated steel punches small round pads of 1-1.5 cm² surface were cut from the three-layer laminate preparations to be tested. The pads contained sub-therapeutic doses of the active ingredient (i.e., about 1/10-1/15 of the amount required for a 24 hour release). After removal of the protective layer, the pads were applied to the inner arm surface of voluntaries and left in site for 24 hours. At the end of the trial the possible presence of any visible reaction on the epidermis was evaluated.

In order to evaluate the results the following arbitrary score scale was employed:
- no inflammation detectable = 0
- detectable inflammation = +
- evident inflammation = ++

The tests were repeated more than once on the same subject, both right after and after 15 days from the first application.

The results so obtained are as follows:
1. Preparations with no ethanolamides:
   inflammation ++ (30%); + (65%); 0 (5%).
2. Preparations containing ethanolamides:
   inflammation ++ (0); + (21%); 0 (79%).

While taking into account the limits of the experimentation and the variability in the sensitivity of the treated subjects, it appears to be confirmed that ethanolamides are endowed with an antiinflammatory and skin protective activity. The beneficial effect of ethanolamides was even more evident for repeated applications.

### EXAMPLE 10

### TTS for the administration of estradiol

Following the procedure of Example 1, matrices with a constant concentration of estradiol (Sigma) were prepared. Some of the said matrices contained oleic diethanolamide (Henkel) as the permeation enhancer, other contained a mixture of oleic diethanolamide and N-(2-hydroxyethyl)acetamide (Aldrich), respectively as enhancer and as absorption assistant. The adhesive employed was Durotak® 387-2052 (National Stark Chemical Co.).

The transdermal fluxes so obtained are expressed in the following table in µg/cm² h, as the average over three tests +/- standard error.

**TABLE 6**

| Transdermal flux of estradiol | | | |
|---|---|---|---|
| Components | Formulation under test | | |
| | n°. 1 | n°. 2 | n°. 3 |
| Estradiol (%) | 2 | 2 | 2 |
| Oleic diethanolamide (%) | 0 | 5 | 5 |
| N-(2-hydroxyethyl)acetamide (%) | 0 | 0 | 15 |
| Adhesive | q.s. to 100 | q.s. to 100 | q.s. to 100 |
| Transdermal flux (µg/cm² h) | 0.25±0.06 | 0.38±0.10 | 0.74±0.08 |

In the formulation No. 3, the percentage of absorption assistant has been raised in order to exploit the co-solvent properties of such compound.

As it may be noted, the preparations containing mixtures of enhancer and absorption assistant according to the invention produced transdermal fluxes of estradiol higher than those of the controls with no penetration enhancers, and also higher than those containing the enhancers without absorption assistants.

### EXAMPLE 11

### TTS for the administration of estradiol

The experimentation of Example 10 was repeated replacing oleic diethanolamide with oleic monoethanolamide (Henkel), and keeping N-(2-hydroxyethyl)acetamide as absorption assistant. Transdermal fluxes of estradiol higher than from the controls with no enhancers have been obtained, and also higher than from the comparative samples containing permeation enhancers with no absorption assistant.

### EXAMPLE 12

### TTS for the administration of estradiol

The experimentation of Example 10 was repeated replacing oleic diethanolamide with lauric diethanolamide (Henkel), and keeping N-(2-hydroxyethyl)acetamide as absorption assistant. Transdermal fluxes of estradiol higher than from the controls with no enhancers have been obtained, and also higher than from the comparative samples containing permeation enhancers with no absorption assistant.

### EXAMPLE 13

### TTS for the administration of estradiol

The experimentation of Example 10 was repeated replacing oleic diethanolamide with palmitic diethanolamide (Henkel), and keeping N-(2-hydroxyethyl)acetamide as absorption assistant. Also in this case, transdermal fluxes of estradiol higher than from the controls with no enhancers have been obtained, and also higher than from the comparative samples containing permeation enhancers with no absorption assistant.

### EXAMPLE 14

### TTS for the administration of estradiol

The experimentation of Example 10 was repeated replacing oleic diethanolamide with undecylenic monoethanolamide (Henkel), and keeping N-(2-hydroxyethyl)acetamide as absorption assistant. Transdermal fluxes of estradiol higher than from the controls with no enhancers have been obtained, and also higher than from the comparative samples containing permeation enhancers with no absorption assistant.

### EXAMPLE 15

### Formulation for the topical administration of methyl nicotinate

Some hydroalcoholic gel formulations containing methyl nicotinate as the active ingredient were produced according to conventional preparation methods. All of the tested samples except the control contained oleic diethanolamide as the permeation enhancer, and two of them also contained different amounts of N-(2-hydroxyethyl)acetamide as absorption assistant. The compositions were applied by spreading them as a thin layer on the exposed skin surface of the experimental apparatus referred to in Example 1. The transdermal fluxes of methyl nicotinate thus measured are shown in the following table.

**TABLE 7**

| Transdermal flux of methyl nicotinate from a hydroalcoholic gel | | | | |
|---|---|---|---|---|
| Components | Formulation under test | | | |
| | n°.1 | n°.2 | n°.3 | n°.4 |
| methyl nicotinate (%) | 0.5 | 0.5 | 0.5 | 0.5 |
| carboxyvinyl polymer (%) | 1 | 1 | 1 | 1 |
| propylene glycol (%) | 16 | 16 | 16 | 16 |
| triethanolamine (%) | 1 | 1 | 1 | 1 |
| ethanol (%) | 10 | 10 | 10 | 10 |
| oleic diethanolamide (%) | 0 | 4 | 4 | 4 |
| N-(2-hydroxyethyl)acetamide (%) | 0 | 0 | 6 | 16 |
| water | q.s to 100 | q.s. to 100 | q.s to 100 | q.s. to 100 |
| transdermal flux (µg/cm² h) | 18.1±0.06 | 26.0±0.09 | 38.3±0.11 | 51.9±0.08 |

The previous data clearly show that oleic diethanolamide as the permeation enhancer and the combination of oleic diethanolamide with N-(2-hydroxyethyl)acetamide as the absorption assistant effectively increase the methyl nicotinate release from the hydroalcoholic gel. N-(2-hydroxyethyl) acetamide, acting as assistant for the absorption of methyl nicotinate and of oleic diethanolamide, also performs, at sufficiently high concentrations, a co-solvent action.

From the foregoing experimental results it appears that the addition of N-ethanolacetamide to the compositions for transdermal and dermal administration containing, as permeation enhancers, fatty acid mono- or dialkanolamides has the following advantages:
- increases the HLB value of the composition, to reach the optimal range of 4-7;
- provides an agent acting as absorption assistant;
- provides an agent acting as co-solvent, allowing in many cases to increase the active ingredient concentration of the formulation;
- increases the antiallergic and antiinflammatory activity of the composition;
- may exert, depending on the features of the active ingredient, a modulating effect on the absorption, particularly useful for the most recent three-layer laminate transdermal devices.

The present invention has been disclosed with particular reference to some specific embodiments thereof, but it should be understood that modifications and changes may be made by the persons skilled in the art without departing from the scope of the invention as defined in the appended claims.

## Claims

1. A composition for the transdermal and dermal administration of biologically active agents comprising an effective amount of one or more biologically active agents, one or more penetration enhancers chosen from the group consisting of monoalkanolamides and dialkanolamides of carboxylic acids having at least 8 carbon atoms, wherein the hydroxyalkyl groups contain from 2 to 21 carbon atoms, one or more absorption assistants chosen from the group consisting of monoethanolamides and diethanolamides of carboxylic acids having from 1 to 4 carbon atoms and, optionally, further biologically acceptable permeation adjuvants and excipients.

2. The composition according to claim 1, wherein said penetration enhancer is a monoalkanolamide or a dialkanolamide of a mono-unsaturated, di-unsaturated or tri-unsaturated aliphatic acid having from 8 to 22 carbon atoms, optionally substituted with from 1 to 3 hydroxy groups.

3. The composition according to claim 2, wherein said monoalkanolamide or dialkanolamide of the penetration enhancer is, respectively, a monoethanolamide or a diethanolamide.

4. The composition according to any one of claims 1-3, wherein said absorption assistant is N-(2-hydroxyethyl)acetamide or N,N-di(2-hydroxyethyl)acetamide.

5. The composition according to any one of claims 1-4 further comprising, as a co-solvent, an aliphatic alcohol chosen from the group consisting of ethanol, n-propanol, isopropanol, buthanol and mixtures thereof.

6. A composition according to any one of claims 1-5 for the transdermal administration of pharmaceutically active agents.

7. The composition according to claim 6, comprising from 0.5% to 40% by weight of said one or more penetration enhancers and from 0.1% to 40% by weight of said one or more absorption assistants.

8. The composition according to claims 6 or 7, presented in a therapeutic transdermal system (TTS).

9. The composition according to claim 8, included in a TTS of the solid matrix type, admixed with one or more suitable pressure sensitive adhesives.

10. The composition according to claim 9, comprising from 2.5% to 15% by weight of said one or more penetration enhancers and from 0.2% to 20% by weight of said one or more absorption assistants.

11. The composition according to any one of claims 6-10, wherein said pharmaceutically active agents are chosen from the group consisting of: antianginal and vasodilating agents, steroids, in particular estrogens and progestogens, and rubefacient and blood flow stimulating agents.

12. A transdermal therapeutic system for administration of pharmaceutically active agents consisting of a laminate comprising:
(a) a backing layer impermeable to the constituents of the adjacent layer (b);
(b) a matrix layer consisting of a solid mixture containing an effective amount of one or more pharmaceutically active agents, one or more penetration enhancers chosen from the group consisting of monoalkanolamides and dialkanolamides of carboxylic acids having at least 8 carbon atoms, wherein the hydroxyalkyl groups contain from 2 to 21 carbon atoms, one or more absorption assistants chosen from the group consisting of monoethanolamides and diethanolamides of carboxylic acids having from 1 to 4 carbon atoms and one or more pressure-sensitive adhesives, as well as further biologically acceptable permeation adjuvants and excipients;
(c) a removable protective layer, to be peeled from said matrix layer before use.

13. The transdermal therapeutic system according to claim 12, wherein said penetration enhancer is a monoethanolamide or a diethanolamide of a mono-unsaturated, di-unsaturated or tri-unsaturated aliphatic acid having from 8 to 22 carbon atoms, optionally substituted with from 1 to 3 hydroxy groups.

14. The transdermal therapeutic system according to claims 12 or 13, wherein said absorption assistant is N-(2-hydroxyethyl)acetamide or N,N-di(2-hydroxyethyl)acetamide.

## Patentansprüche

1. Zusammensetzung zur transdermalen und dermalen Verabreichung von biogisch wirksamen Stoffen, welche eine effektive Menge eines oder mehreren biologisch wirksamen Stoffen, einen oder mehrere Penetration-Hilfsstoffe, die von einer aus Monoalkanolamiden und Dialkanolamiden von Karboxylsäuren mit mindestens 8 Kohlenstoffatomen bestehenden Gruppe ausgewählt sind, enthält, worin die Hydroxyalkylgruppe 2 bis 21 Kohlenstoffatome bestehen, einen oder mehrere Absorption-Hilfsstoffe, die von einer aus Monoäthanolamiden und Diäthanolamiden der Karboxylsäuren mit 1 bis 4 Kohlenstoffatomen bestehenden Gruppe ausgewählt sind, und wahlweise andere biologisch verträgbare Permeation-Hilfstoffe und Wirkstoffträger enthält.

2. Zusammensetzung nach Anspruch 1, worin der vorgenannte Penetration-Hilfsstoff ein Monoalkanolamid oder Dialkanolamid einer mono-, di- oder tri-ungesätigter aliphatischen Säure mit 8 bis 22 Kohlenstoffatomen ist, die wahlweise durch 1 bis 3 Hydroxygruppen ersetzbar ist.

3. Zusammensetzung nach Anspruch 2, worin das vorgenannte Monoalkanolamid oder Dialkanolamid des Penetration-Hilfsstoffes ein Monoäthanolamid bzws. ein Diäthanolamid ist.

4. Zusammensetzung nach je einem der Ansprüche 1 bis 3, worin der vorgenannte Absorption-Hilfsstoff N-(2-Hydroxyäthyl)-Acetamid oder N,N-Di(2-Hydroxyäthyl)-Acetamid ist.

5. Zusammensetzung nach je einem der Ansprüche 1 bis 4, welche ausserdem, als ein Hilfs-Lösungsmittel, einen aliphatischen Alcohol enthält, der von einer aus Äthanol, n-Propanol, Isopropanol, Buthanol und deren Mischungen bestehenden Gruppe ausgewählt ist.

6. Zusammensetzung nach je einem der Ansprüche 1 bis 5 für die transdermale Verabreichung von pharmazeutisch wirksamen Stoffen.

7. Zusammensetzung nach Anspruch 6, welche 0,5 bis 40 Gew. % des vorgenannten eines oder mehreren Penetration-Hilfsstoffen und 0,1 bis 40 Gew. % des vorgenanten eines oder mehreren Absorption-Hilfsstoffen enthält.

8. Zusammensetzung nach Anspruch 6 oder 7, welche in einem therapeutisch transdermalen System (TTS) vorgeführt ist.

9. Zusammensetzung nach Anspruch 8, welche in einem TTS der fest-Matrix-Art enthalten ist, das mit einem oder mehreren angebrachten druckempfindlichen Klebstoff vermisch ist.

10. Zusammensetzung nach Anspruch 9, welche 2,5 bis Gew. 15% des vorgenanten eines oder mehrerer Penetration-Hilfsstoffe und 0,2 bis 20 Gew. % des vorgenannten eines oder mehrer Absorption-Hilfsstoffe enthält.

11. Zusammensetzung nach je einem der Ansprüche 6 bis 10, worin die vorgenannten pharmazeutisch wirksame Wirkstoffe von einer aus antianginal und vasodilatatorisch wirksamen Stoffen, Steroiden, insbesondere Östrogenen und Progestogenen, und rötender und Blutfluss anregenden Stoffen bestehenden Gruppe ausgewählt sind.

12. Therapeutisches Transdermalsystem zur Verabreichung von pharmazeutisch wiksamen Stoffen bestehend aus einem Laminat, das folgendermassen aufgebaut ist:
(a) eine gegenüber den Bestandteilen der angrenzenden Schicht (b) undurchlässige Schicht;
(b) eine Matrixschicht aus einem festen Gemisch, das eine effektive Menge eines oder mehreren pharmazeutisch wirksamen Stoffe enthält, einen oder mehrere Penetration-Hilfsstoffe, die von einer aus Monoalknolamiden und Dialkanolamiden von Karboxylsäuren mit mindesten 8 Kohlenstoffatomen bestehenden Gruppe ausgewählt sind, worin die Hydroxyalkylgruppen 2 bis 21 Kohlenstoffatome bestehen, einen oder mehrere Absorption-Hilfsstoffe, die von der aus Monoäthanolamiden und Diäthanolamiden mit 1 bis 4 Kohlenstoffeatomen bestehenden Gruppe ausgewählt sind, und einen oder mehrere druckempfindliche Klebstoffe, sowie andere biologisch verträgbare Permeation-Hilfstoffe und Wirkstoffträger enthalten;
(c) eine abnehmbar Schutzschichte, die vor dem Gebrauch von der vorgenannten Matrizeschicht abgetrennt wird.

13. Therapeutisches Transdermalsystem nach Anspruch 12, worin der vorgenannte Penetration-Hilfsstoff ein Monoäthanolamid oder ein Diäthanolamid einer mono- oder di- oder tri-ungesättiger aliphatischen Säure mit 8 bis 22 Kohlenstoffatomen ist, die wahlweise durch 1 bis 3 Hydroxygruppen ersetzbar ist.

14. Therapeutisches Transdermalsystem nach Anspruch 12 oder 13, worin der vorgenannte Absorption-Hilfsstoff ein N-(2-Hydroxyäthyl)-Acetamid oder ein N,N-Di(2-Hydroxyäythyl)-Acetamid ist.

## Revendications

1. Composition pour l'admnistration transcutanée et cutanée d'agents biologiquement actifs, comprenant une quantité efficace d'un ou plusieurs agents biologiquement actifs, un ou plusieurs promoteurs de pénétration choisis du groupe consistant de monoalcanolamides ou dialcanolamides d'acides carboxyliques ayant au moins 8 atomes de carbone, dans lesquels groupes hydroxyalcoyle contiennent de 2 à 21 atomes de carbone, un ou plusieurs assistants d'absorption choisis du groupe consistant de monoéthanolamides et diéthanolamides d'acides carboxyliques ayant de 1 à 4 atomes de carbone, et facultativement d'autres coadjuvants de perméation et excipients biologiquement acceptables.

2. Composition selon la revendication 1, dans laquelle ledit promoteur de pénétration est un monoalcanolamide ou un dialcanolamide d'un acide aliphatique mono-, di- ou tri-insaturé ayant de 8 à 22 atomes de carbone, facultativement substitué par 1 à 3 groupes hydroxyle.

3. Composition selon la revendication 2, dans laquelle ledit monoalcanolamide ou dialcanolamide du promoteur de pénétration est, respectivement, un monoéthanolamide ou un diéthanolamide.

4. Composition selon une quelconque des revendications 1 à 3, dans laquelle ledit assistant d'absorption est N-(2-hydroxyéthyl)acétamide ou N,N-di(2-hydroxyéthyl)acétamide.

5. Composition selon une quelconque des revendications 1 à 4, comprenant en outre, comme un co-solvant, un alcohol aliphatique choisi du groupe consistant d'éthanol, n-propanol, isopropanol, buthanol et leur mélanges.

6. Composition selon une quelconque des revendications 1 à 5 pour l'administration transcutanée d'agents pharmaceutiquement actifs.

7. Composition selon la revedication 6, comprenant 0,5-40% en poids dudit un ou plusieurs promoteurs de pénétration et 0,1-40% en poids dudit un ou plusieur assistants d'absorption.

8. Composition selon la revendication 6 ou 7, présentée dans un système transdermique thérapeutique (TTS).

9. Composition selon la revendication 8, comprise dans un TTS du type à matrice solide, mélangée avec un ou plusieurs moyens adhésifs appropriés, sensible à pression.

10. Composition selon la revendication 9, comprenant 2,5-15% en poids dudit un ou plusieurs promoteurs de pénétration et 0,2-20% en poids dudit un ou plusieurs assistants d'absorbtion.

11. Composition selon une quelconque des revendications 6 à 10, dans laquelle lesdits agents pharmaceutiquement actifs sont choisis du groupe consistant de agents antianginales et vaso-dilatateurs, stéroides, particulièrement aestrogènes et progestogènes et agents rubéfiants et stimulants la circulation du sang.

12. Système transdermique thérapeutique pour l'administration d'agents pharmaceutiquement actifs consistant d'un matériel laminé comprenant:
(a) une couche de support imperméable aux constituants de la couche adjacente (b);
(b) une couche matrice consitant d'un mélange solide contenant une quantité efficace d'un ou de plusieurs promoteurs de pénétration choisis du groupe consistant de monoalcanolamides ou dialcanolamides d'acides carboxyliques ayant au moins 8 atomes de carbone, dans lesquels les groupes hydroxyalcoyles ont 2 à 21 atomes de carbone, un ou plusieur assistant d'absorbtion choisis du groupe consistant de monoétanolamides et diéthanolamides des acides carboxyliques ayant 1 à 4 atomes de carbone et un ou plusieurs moyens adhésifs sesibles à pression ainsi que coadjuvants de perméation et excipients biologiquement acceptables;
(c) une couche de protection amovible, qui doit être enlevée de ladite couche matrice avant de l'usage.

13. Système transdermique thérapeutique selon la revendication 12, dans lequel ledit promoteur de pénétration est un monoéthanolamide ou un diéthanolamide d'un acide aliphatique acide aliphatique mono-, di- ou tri-insaturé ayant de 8 à 22 atomes de carbone, facultativement substitué par 1 à 3 groupes hydroxyle.

14. Système transdermique thérapeutique selon les revendications 12 ou 13, dans lequel ledit assistant d'asorption est N-(2-hydroxéthyl)acétamide ou N,N-di(2-hydroxyéthyl)acétamide.
